# EUROPEAN PATENT APPLICATION

(11) **EP 3 435 301 A1**
(43) Date of publication of application: **30.01.2019**
(21) Application number: 18179763.0
(22) Date of filing: 26.06.2018
(51) Int. Cl.: G06Q 10/06

(54) **SYSTEM FOR PROVIDING AIR QUALITY INFORMATION**

(30) Priority: 27.07.2017 TW 106125337
(71) Applicant: Microjet Technology Co., Ltd, Hsinchu (TW)
(72) Inventor: Mou, Hao-Jan, Hsinchu (TW); Hsueh, Ta-Wei, Hsinchu (TW); Mo, Li-Pang, Hsinchu (TW); Chen, Shih-Chang, Hsinchu (TW); Lin, Ching-Sung, Hsinchu (TW); Huang, Chi-Feng, Hsinchu (TW); Han, Yung-Lung, Hsinchu (TW); Chen, Hsuan-Kai, Hsinchu (TW)
(74) Representative: Uexküll & Stolberg

(57) **Abstract**

A system (100) for providing an air quality information (AI) is disclosed. The system (100) includes plural mobile devices (1a, 1b, 1c), a cloud data processing device (2) and a client device (3). Each mobile device (1a, 1b, 1c) has a positioning module (12) and an actuating and sensing module (13) to generate a single-point air quality data (SIa, SIb, SIc) to transmit to the cloud data processing device (2). The cloud data processing device (2) collects the plural single-point air quality data (SIa, SIb, SIc) transmitted from the plural mobile devices (1a, 1b, 1c), and combines these data with a map data (211) and a meteorological data (212) to generate an instant air quality map (21). When the cloud data processing device (2) receives a current location data (CL) transmitted from the client device (3), an information (AI) is processed based on the instant air quality map (21) and the current location data (CL), so as to transmit the information (AI) including a motion direction, a designated route, an air quality information, an abnormal-air-quality notification or an evacuated route for the client device (3).

## Description

### FIELD OF THE INVENTION

The present disclosure relates to a data processing system for a specific application, and more particularly to a system of collecting air sensing data from a plurality of mobile devices by a cloud apparatus and computing to generate an air quality information for providing the air quality information.

### BACKGROUND OF THE INVENTION

Nowadays, the air pollution problems are becoming increasingly serious in our country and its neighboring regions. In particular, the concentration of fine suspended particles (PM 2.5) is often too high, and the public gradually develops habits of accessing real-time air quality monitoring data online at any time in daily life, so as to make immediate protective measures against air pollution. Taking the air quality monitoring network of the Environmental Protection Agency of the Executive Yuan as an example, the current air quality monitoring system utilizes the air quality monitoring stations established throughout the country to sample and analyze the air, and the monitored data from the fixed-point monitoring stations are integrated as an air quality index (AQI) to quantitatively describe the air quality and be published on the website for public inspection.

However, the fixed-point monitoring method includes the following disadvantages. Firstly, since the construction cost of the monitoring stations is very expensive, the installation number of the monitoring stations is limited. The fixed-point monitoring stations can provide the air quality measured at the specific locations and the place surrounding the specific area merely, instead of the entire area covering all users' locations completely. Furthermore, the users cannot obtain the accurate air quality data based on their own locations. In addition, when the AQI index of a specific area reaches a level representing harmful to human health, the air quality monitoring network only provides the users of the area with a suggestion to avoid going out, but does not provide further useful information to aid the users to deal with the poor air quality.

In order to overcome the drawbacks of the fixed-point monitoring method for the air quality, Taiwan patent application with the publication number TW 201719540 discloses a cloud sharing method for positioning and air detection, wherein a handheld mobile device is combined with an air sensing unit. In this way, the handheld mobile device can be used to position a specific location and simultaneously sense the air quality data at the specific location. Then, the handheld mobile device uploads the results of the positioning and the air detection to a cloud data processing platform and marks it on a social platform. The cloud data processing platform compares and analyzes the measured air quality data with the air database. If the analyzed results indicate that the measured air quality fails to meet the standard, the cloud data processing platform will send out a message to inform the relevant units to carry out maintenance and provide the analyzed results to other users for reference.

However, the above methods only deal with the single information generated by the individual handheld mobile devices, but does not integrate the air quality data sensed by the plural handheld mobile devices at different locations. Furthermore, the air quality data is not merged with the other types of data information to generate more valuable derivative information for users' reference. At the same time, the method of the patent application evaluate the level of air quality merely, but does not specify other possible forms and contents of the analyzed results. In addition, there is no practical embodiment or structure of the air sensing unit mentioned in the specification of the patent application.

Therefore, there is a need of providing a system for providing air quality information to solve the drawbacks in prior arts.

### SUMMARY OF THE INVENTION

Since the current air quality monitoring systems sample and sense the air quality through fixed-point stations, the sensed air quality data cannot include information of the air quality exactly at all users' locations. When the handheld mobile devices and the air sensing units are combined and utilized according to the conventional technologies, the air quality can be sensed at anytime and anywhere, but there is a lack of integration and utilization of the air quality data at different time and different place. There is no relevant data combined to generate the derivative information with additional benefits for users. Moreover, the users cannot actively search for the air quality information at specific locations. Therefore, the prior art cannot effectively exert the value of the air quality data sensed by the handheld mobile devices. In addition, the prior art does not tend to improve the air sensing unit. Therefore, when the air sensing unit is applied to a handheld mobile device and sensed on a motion, the accuracy of the sensing result is really questionable.

In order to solve the above problems, the present disclosure provides a plurality of mobile devices with actuating and sensing modules to sense a plurality of single-point air quality data at their respective locations and the sensed single-point air quality data are transmitted to a cloud data processing device. The cloud data processing device collects the single-point air quality data of the mobile devices at a predetermined time, processes the single-point air quality data and generates an instant air quality map by combining the processed single-point air quality data with a map data and a meteorology data. At this time, a client device can transmit a current location data of the current location to the cloud data processing device through a communication transmission path and can send a request for information from the cloud data processing device. The cloud data processing device generates the information based on the instant air quality map and the current location data of the current location, and transmits the information to the client device.

In contrast to the prior art, the present disclosure provides a system combining the air quality data from a plurality of mobile devices in series and integrating other relevant data into the instant air quality map. In this way, it not only makes full use of the advantages of the number and mobility of the mobile devices, but also makes the information more accurate than that of the conventional fixed-point air monitoring system. In addition, since the processed single-point air quality data of the present disclosure are further combined with the map data and the meteorology data, various derivative information beneficial to the user can be generated. The derivative information includes a designated route, air quality information, an abnormal-air-quality notification, a warning notification, or an evacuation route. As to the prior art, the air quality information are determined by simple data from fixed point locations, and it fails to provide users with the ability to actively query the air quality at a specific location. Therefore, the present disclosure further utilizes the information for big data operations at a higher level and it facilitates to improve the benefits significantly.

In accordance with an aspect of the present disclosure, there is provided a system for providing an air quality information. The system includes a plurality of mobile devices, a cloud data processing device and a client device. Each mobile device includes a positioning module and an actuating and sensing module. The actuating and sensing module includes an actuating device and a sensor. The actuating device introduces air from the external environment into the inner of the actuating and sensing module, and the sensor senses the air to generate the air-sensing data. The positioning module of the mobile device generates a position data. Each mobile device generates a single-point air quality data for its location at a predetermined time by computing and combining the air-sensing data and the position data from the actuating and sensing module. In contrast to the prior art, since the mobile device of the present disclosure provides the actuating and sensing module to actuate the air from the external environment into the inner for sensing, it facilities the mobile device to provide a better adaptability of the moving situation, and it ensures that the air quality can be sensed accurately while the mobile device is moving.

The above contents of the present disclosure will become more readily apparent to those ordinarily skilled in the art after reviewing the following detailed description and accompanying drawings, in which:

### BRIEF DESCRIPTION OF THE DRAWINGS

FIG. 1 is a block diagram illustrating a system for providing an air quality information according to an embodiment of the present disclosure;
FIG. 2 is a flow chart illustrating a method of providing an air quality information by implementing the system according to an embodiment of the present disclosure;
FIG. 3 is a flow chart illustrating a method of providing an air quality information and a designated route by implementing the system according to an embodiment of the present disclosure; and
FIG. 4 is a flow chart illustrating a method of providing an air quality information and a warning notification by implementing the system according to an embodiment of the present disclosure.

### DETAILED DESCRIPTION OF THE PREFERRED EMBODIMENT

The present disclosure will now be described more specifically with reference to the following embodiments. It is to be noted that the following descriptions of preferred embodiments of this invention are presented herein for purpose of illustration and description only. It is not intended to be exhaustive or to be limited to the precise form disclosed.

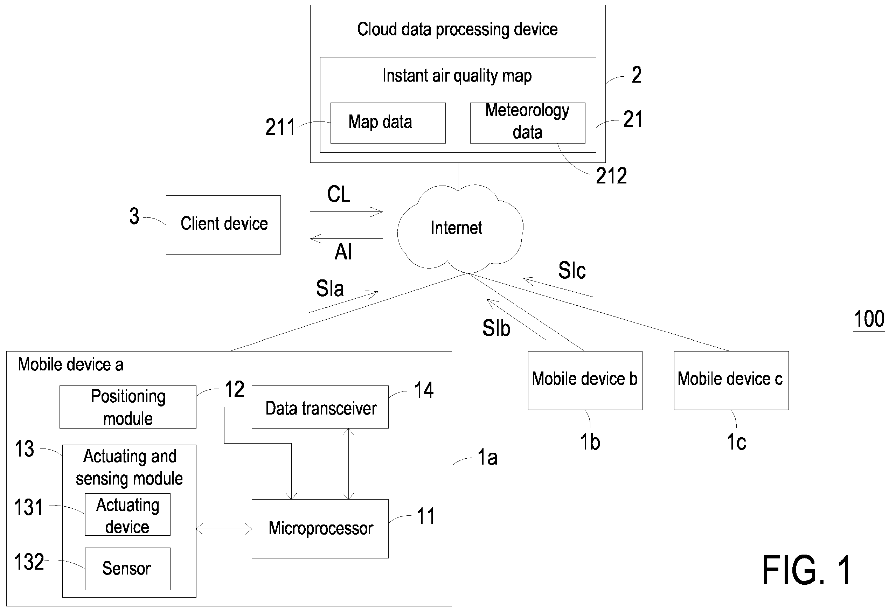

Please refer to FIG. 1. The present discourse provides a system 100 for providing an air quality information including at least one positioning module 12, at least one position data, at least one actuating and sensing module 13, at least one air-sensing data, at least one predetermined time, at least one single-point air quality data, at least one cloud data processing device 2, at least one instant air quality map, at least one client device 3, at least one current location data and at least one information. The number of the positioning module 12, the position data, the actuating and sensing module 13, the air-sensing data, the predetermined time, the single-point air quality data, the cloud data processing device 2, the instant air quality map, the client device 3, the current location data and the information is exemplified by one for each in the following embodiments but not limited thereto. It is noted that each of the positioning module 12, the position data, the actuating and sensing module 13, the air-sensing data, the predetermined time, the single-point air quality data, the cloud data processing device 2, the instant air quality map, the client device 3, the current location data and the information can also be provided in plural numbers.

Please refer to FIG. 1, which is a block diagram illustrating a system for providing an air quality information according to an embodiment of the present disclosure. The system 100 for providing the air quality information includes a plurality of mobile devices 1a, 1b and 1c, a cloud data processing device 2 and a client device 3. The mobile devices 1a, 1b and 1c have the same structure and can be for example but not limited to a mobile phone, a tablet, a wearable device, or any similar mobile electronic device constructed to contain microprocessors, RAM, and other components. The mobile device 1a is taken as an example for further describing the structure of the mobile device 1a, 1b and 1c as follows. As shown in FIG. 1, the mobile device 1a includes a microprocessor 11, a positioning module 12, an actuating and sensing module 13 and a data transceiver 14. The microprocessor 11 is electrically connected to the positioning module 12, the actuating and sensing module 13 and the data transceiver 14. The positioning module 12 can be a GPS satellite positioning module, but not limited thereto.

The actuating and sensing module 13 includes an actuating device 131 and a sensor 132. The actuating device 131 is a driver capable of driving the desired system in response to a control signal. The function of the actuating device 131 is to drive air from the external environment, so that the air is introduced into the interior of the actuating and sensing module 13. The actuating device 131 can include an electric actuator, a magnetic actuator, a thermal actuator, a piezoelectric actuator, and a fluid actuator. For example, it can be an electric actuator such as an AC-DC motor or a stepping motor, a magnetic actuator such as a magnetic coil motor, a thermal actuator such as a heat pump, a piezoelectric actuator such as a piezoelectric pump, or a fluid actuator such as a gas pump and a liquid pump, but is not limited thereto.

The sensor 132 is disposed adjacent to the actuating device 131, so that at least one target in the air (which is introduced by the actuating device 131) can be sensed and a corresponding air-sensing data is generated. The sensor 132 can include a sensor such as a temperature sensor, a volatile organic compound sensor (for example, a sensor for sensing the formaldehyde and the ammonia), a particulate sensor (for example, a PM 2.5 particle sensor), a carbon monoxide sensor, a carbon dioxide sensor, an oxygen sensor, an ozone sensor, other gas sensors, a humidity sensor, a moisture sensor, a measuring sensor used for measuring the compounds and/or biological substances in water, other liquids or air (for example, a water quality sensor), other liquid sensors, a light sensor used for measuring the environment, or a group formed by any combination of the sensors described above, but is not limited thereto. Therefore, the target detected by the sensor 132 can be a volatile organic gas such as ammonia or ethanol, or at least one of carbon monoxide, carbon dioxide, sulfur dioxide, nitrogen dioxide, a suspended particle, a fine suspended particle, oxygen, ozone or any combination thereof. The sensor 132 can also sense a virus, a bacterium or a microorganism by a direct or indirect method, but is not limited thereto.

The client device 3 can be a mobile phone, a tablet computer or a wearable device, which includes a GPS satellite positioning function and a communication transmission module, or can be any mobile electronic device, which is constructed to include components such as a microprocessor and a RAM, but is not limited thereto. In some embodiments, the client device 3 can also be one of the plurality of mobile devices 1a, 1b and 1c.

The cloud data processing device 2 is a computer or any similar device constructed to include CPU, RAM, and etc., and have a data analysis management function. In the system 100, the cloud data processing device 2 serves as a server to connect the mobile device 1a, 1b and 1c and the client device 3 through the internet, so as to transmit and receive the information through a wired or wireless communication transmission path. The wired communication transmission path can be established by utilizing a RS485 communication port, an RS232 communication port, a Modbus communication port or a KNX communication port. The wireless communication transmission path can be established by utilizing a Zigbee communication technology, a Z-wave communication technology, an RF communication technology, a Bluetooth communication technology, a Wifi communication technology or an EnOcean communication technology. The data transceiver 14 of the mobile device 1a can also be a module, which utilizes various communication transmission technologies described above.

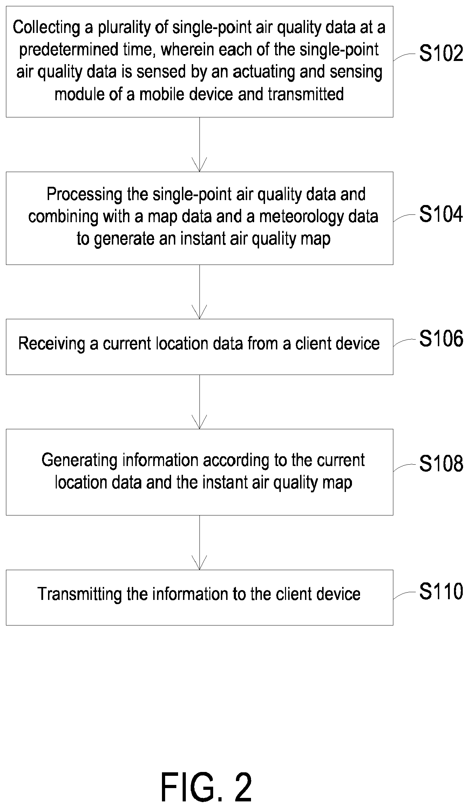

Please refer to FIGS 1 and 2. FIG. 2 is a flow chart illustrating a method of providing an air quality information by implementing the system according to an embodiment of the present disclosure. In the embodiment, at the step S102, the cloud data processing device 2 collects a plurality of single-point air quality data SIa, SIb and SIc at a predetermined time. The cloud data processing device 2 can perform a collection operation at regular intervals, for example, 5 minutes or 1 hour. The single-point air quality data SIa, SIb and SIc are generated by the mobile devices 1a, 1b and 1c respectively at the predetermined time. Taking the mobile device 1a as an example, the user can pre-set the positioning module 12 to automatically generate a position data at the predetermined time, or actively request the positioning module 12 to generate the position data at the predetermined time. The position data can be a coordinate of a location of the mobile device 1a positioned by the GPS satellite positioning system and includes a timestamp. While the position data is generated, the actuating and sensing module 13 of the mobile device 1a synchronously is operated to inhale the air from the external environment and to sense the air to generate the air-sensing data. The microprocessor 11 receives the position data from the positioning module 12 and the air-sensing data from the actuating and sensing module 13 respectively, and computes the single-point air quality data SIa. The single-point air quality data SIa maintains the timestamp corresponding to the position data to record the coordinate of the location and the air-sensing data from the location at the specific time. The data transceiver 14 receives the single-point air quality data SIa from the microprocessor 11 and transmits it to the cloud data processing device 2 through the communication transmission path.

At the step S104, the cloud data processing device 2 integrates and processes the single-point air quality data SIa, SIb and SIc transmitted from the mobile devices 1a, 1b and 1c. The single-point air quality data SIa, SIb, and SIc can be the data generated by the mobile devices 1a, 1b, and 1c at all predetermined times during the regular interval of the collection operation of the cloud data processing device 2. Namely, if the collection operation of the cloud data processing device 2 is performed every 10 minutes and time points recorded by the timestamps of the single-point air quality data SIa, SIb and SIc are at a 10-minute segment ranged between the time of the previous collection operation and the time of the subsequent upcoming collection operation, the single-point air quality data SIa, SIb and Sic are regarded as a batch of air-sensing data at the same time segment and processed together by the cloud data processing device 2.

The cloud data processing device 2 combines the processed air-sensing data with a map data 211 (Geographic information) to generate an instant air quality map 21, and the instant air quality map 21 can present all the acquired single-point air quality data SIa, Sib and SIc in the time segment. Further, the cloud data processing device 2 may also connect to a meteorological center to obtain an instant meteorology data 212. The meteorology data 212 may be further taken into consideration when combining the processed air-sensing data, thereby generating another updated instant quality map 21. The meteorology data can include at least one selected from the group consisting of a wind direction, a wind speed, a humidity, a temperature, a weather pattern and a combination thereof. Accordingly, if there is no corresponding air quality information at a specific location, the average of the air quality information of the other plural locations closest to the specific location is processed into a result presented as the air quality information of the specific location by utilizing the meteorology data 212 as a parameter. When the number of mobile devices 1a, 1b, and 1c reaches a certain scale, the instant air quality map 21 can provide much higher precision and more widespread than the conventional fixed-point monitoring stations through the big data operations of the cloud data processing device 2.

At the step S106, the client device 3 generates a current location data CL represented a current location thereof. The user can download a mobile application (hereinafter abbreviated as APP). The APP requires the user to enable the data access permission of the GPS positioning module of the client device 3. If the user agrees with the request, a coordinate of a location sensed by the GPS positioning module is automatically designated as the current location data CL and uploaded to the cloud data processing device 2 when the client device 3 is turned on or the APP is executed. Alternatively, when being operated, the APP can accept the user's instructions to generate a current location data CL and upload the current data CL to the cloud data processing device 2. In this way, the current location data CL can represent a GPS coordinate of a location where the client device 3 is located, or a specific location (where is not the location of the client device 3) inputted by the user.

At the step S108, the cloud data processing device 2 receives the current location data CL, and generates an information AI based on the instant air quality map 21 and the current location data CL. The information AI can be air quality information of the current location data CL including for example a concentration of the pollutants of suspended particles, but not limited thereto. At the step S110, the cloud data processing device 2 transmits the air quality information to the client device 3. The client device 3 displays the air quality information on the display (not shown) through the human-machine interface designed for the user to view.

In another embodiment of the present disclosure, the current location of the step S106 can be a GPS coordinate of a location where the client device 3 is located, and the information AI generated based on the instant air quality map and the current location data CL of the step S108 further includes a motion direction. The client device 3 displays the motion direction on the display (not shown) through the human-machine interface designed for the user to view, thereby informing the user about the direction with the good air quality as a recommended reference for the daily schedule.

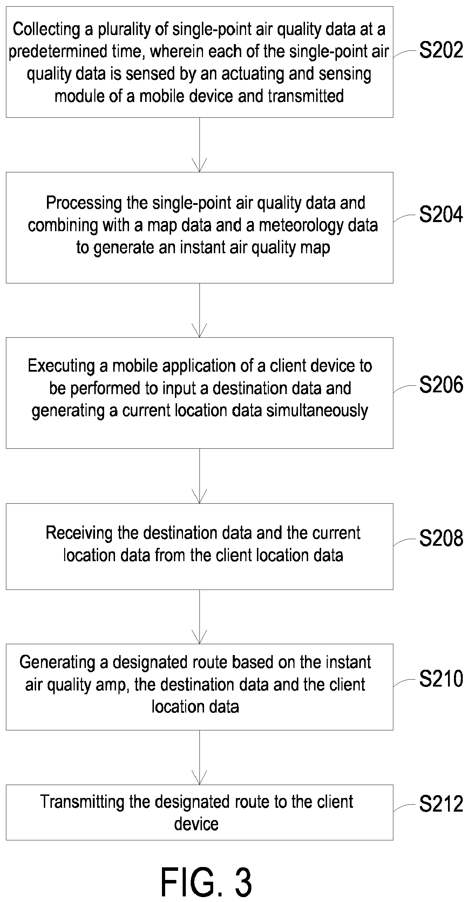

Please refer to FIGS. 1 and 3. FIG. 3 is a flow chart illustrating a method of providing an air quality information and a designated route by implementing the system according to an embodiment of the present disclosure. In this embodiment, the steps S202 and S204 are similar to the steps S102 and S104 of the previous embodiment, and will not be redundantly described herein. However, at the step S206, the client device 3 starts the pre-installed APP and a destination data is inputted by the user. At the same time, the client device 3 detects the GPS coordinate of a location thereof to generate the current location data CL. At the step S208, the cloud data processing device 2 receives the destination data and the current location data CL uploaded by the client device 3 through the communication transmission path. At the step S210, the cloud data processing device 2 generates a designated route by processing the instant air quality map 21 according to the current location data CL and the destination data. The designated route is one path from the current location represented by the current location data CL toward the destination represented by the destination data. At the step S212, the cloud data processing device 2 transmits the designated route to the client device 3 through the communication transmission path, and displays the designated route on the display (not shown) through the human-machine interface designed for the user to view. With the steps described above, the cloud data processing device 2 can generate the designated route based on the updated instant air quality map 21 formed by integrating the meteorology data (e.g., a wind direction or a weather pattern) with the collected single-point air quality data SIa, SIb and SIc, thereby instructing the user to avoid the areas where the air quality may be poor to reach their desired destination.

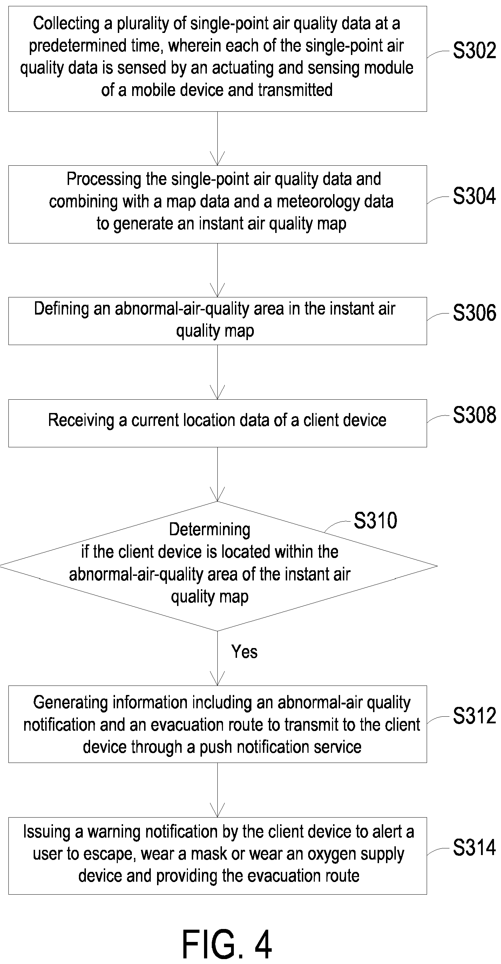

Please refer to FIGS. 1 and 4. FIG. 4 is a flow chart illustrating a method of providing an air quality information and a warning notification by implementing the system according to an embodiment of the present disclosure. In this embodiment, the steps S302 and S304 are similar to the steps S102 and S104 of the previous embodiment, and will not be redundantly described herein. At the step S306, the cloud data processing device 2 further defines at least one abnormal-air-quality area in the instant air quality map 21. The abnormal-air-quality area may be, for example, in a shape of a circle, centered on the location of the pollution source, and bounded by an area where the air quality is inferior to a standard value. At the step S308, the cloud data processing device 2 receives a current location data CL transmitted by a client device 3. The current location data CL is the GPS coordinate of a location where the client device 3 is located, and is automatically generated and automatically uploaded to the cloud data processing device 2 by presetting.

At the step S310, the cloud data processing device 2 determines whether the current location represented by the current location data CL falls within the range of the abnormal-air-quality area. If it is within the range of the abnormal-air-quality area, at the step S312, the cloud data processing device 2 generates an abnormal-air-quality notification. At the step S312, the cloud data processing device 2 actively transmits the abnormal-air-quality notification to the client device 3 through a push notification service. At the step S314, the client device 3 issues a warning notification based on the abnormal-air-quality notification. The warning notification can be for example in any form of visual cues, auditory cues or vibration touch, to alert the user that the air quality of the current location is poor and have to avoid. With the above steps, an escape warning effect can be achieved. For example, carbon monoxide is colorless and odorless. Once the concentration of the carbon monoxide reaches 35ppm in the air, it will cause damage to the human body and may even be fatal. With the method by implementing the system 100 of the present disclosure, the user can be warned to immediately avoid the current location, thereby avoiding the harmful gas.

In some embodiments, at the step S312, the cloud data processing device 2 may further perform a calculation based on the instant air quality map 21 corresponding to the user's current location data CL to generate at least one evacuation route. The evacuation route represents a path from the current location represented by the current location data CL toward an evacuation site. The evacuation site is out of the range of the abnormal-air-quality area and there is a closest traffic distance from the current location of the current location data CL to the evacuation site. At the step S314, the cloud data processing device 2 actively transmits the abnormal-air-quality notification and the evacuation route to the client device 3 through a push notification service. The client device 3 displays the evacuation route on a display (not shown) through a human-machine interface designed for the user to view. With the above steps, the cloud data processing device 2 generates the evacuation route based on the updated instant air quality map 21 formed by integrating the meteorology data (e.g., a wind direction or a weather pattern) with the collected single-point air data SIa, SIb, and Sic. Thus, the system 100 of the present disclosure can instruct the user to leave the area contaminated by harmful gas or heavy smoke in the fire as soon as possible, and has the function to provide the escape instructions away from the public accident.

In other embodiments, at the step S314, the warning notification alerts the user to wear a mask or alerts the user to wear an oxygen supply device, such as an oxygen mask connected to an oxygen bottle.

## Claims

1. A system for providing an air quality information, comprising:
a plurality of mobile devices (1a, 1b, 1c), wherein each of the mobile devices (1a, 1b, 1c) comprises:
a positioning module (12) generating a position data; and
an actuating and sensing module (13) generating at least one air-sensing data;
wherein each of the mobile devices (1a, 1b, 1c) generates a single-point air quality data (SIa, SIb, SIc) at a predetermined time, and the single-point air quality data (SIa, SIb, SIc) includes the position data and the air-sensing data;
a cloud data processing device (2) receiving the single-point air quality data (SIa, SIb, SIc) transmitted from each of the mobile devices (1a, 1b, 1c) through a communication transmission path, processing the single-point air quality data (SIa, SIb, SIc) of the mobile devices (1a, 1b, 1c), and generating an instant air quality map (21) by combining the processed single-point air quality data (SIa, SIb, SIc) with a map data (211); and
a client device (3) generating a current location data (CL) represented a current location to transmit to the cloud data processing device (2) through the communication transmission path and sending a request for information (AI) to the cloud data processing device (2), wherein the cloud data processing device (2) generates the information (AI) according to the current location data (CL) and the instant air quality map (21) and transmits the information (AI) to the client device (3).

2. The system according to claim 1, wherein the actuating and sensing module comprises:
an actuating device (131) drives air from an external environment; and
a sensor (132) disposed nearby the actuating device (131) to sense the air to generate the air-sensing data.

3. The system according to claim 1, wherein each of the mobile devices (1a, 1b, 1c) comprises:
a microprocessor (11) electrically connected with the positioning module (12) and the actuating and sensing module (13) to receive the positioning data and the air-sensing data, and generate the single-point air quality data (SIa, SIb, SIc) by processing the positioning data and the air-sensing data; and
a data transceiver (14) electrically connected with the microprocessor (11) to receive the single-point air quality data (SIa, SIb, SIc) and transmit the single-point air quality data (SIa, SIb, SIc) to the cloud data processing device (2) through the communication transmission path.

4. The system according to claim 1, wherein the air-sensing data is configured to sense at least one selected from the group consisting of carbon monoxide, carbon dioxide, sulfur dioxide, nitrogen dioxide, a suspended particle, a fine suspended particle, oxygen, ozone, a virus, a bacterium, a microorganism and a volatile organic compound.

5. The system according to claim 4, wherein the volatile organic compound is ammonia or ethanol.

6. The system according to claim 1, wherein the information (AI) comprises a motion direction.

7. The system according to claim 1, wherein the client device (3) comprises a mobile application installed therein to be performed to input a destination data, wherein the client device transmits the destination data to the cloud data processing device (2) through the communication transmission path and the cloud data processing device (2) generates the information (AI) based on the instant air quality map (21), the current location data (CL) and the destination data, wherein the information (AI) comprises at least one designated route.

8. The system according to claim 1, wherein the information (AI) is the air quality information comprising the instant air quality map (21) corresponding to the current location data (CL).

9. The system according to claim 1, wherein the cloud data processing device (2) combines the processed single-point air quality data (SIa, SIb, SIc) with a meteorology data (212) to generate an updated instant air quality map, wherein the meteorology data (212) comprises at least one selected from the group consisting of a wind direction, a wind speed, a humidity, a temperature and a weather pattern, wherein the information (AI) comprises a motion direction.

10. The system according to claim 9, wherein the client device (3) comprises a mobile application installed therein to be performed to input a destination data, wherein the client device (3) transmits the destination data to the cloud data processing device (2) through the communication transmission path and the cloud data processing device (2) generates the information (AI) based on the instant air quality map (21) updated, the current location data (CL) and the destination data, wherein the information (AI) comprises at least one designated route.

11. The system according to claim 9, wherein the information (AI) is the air quality information comprising the instant air quality map (21) updated corresponding to the current location data (CL).

12. The system according to claim 1, wherein the information (AI) is transmitted through a push notification service.

13. The system according to claim 12, wherein the cloud data processing device (2) defines at least one abnormal-air-quality area, and while the cloud data processing device (2) determines that the client device (3) is located within the abnormal-air-quality area according to the current location data (CL) transmitted from the client device (3), the client device (3) receives the information (AI) comprising an abnormal-air-quality notification from the cloud data processing device (2) and the client device (3) issues a warning notification based on the abnormal-air-quality notification.

14. The system according to claim 13, wherein the information (AI) comprises an evacuation route, the evacuation route represents a path from the current location toward an evacuation site, and the evacuation site is out of the abnormal-air-quality area.

15. The system according to claim 13, wherein the warning notification alerts a user to wear at least one selected from the group consisting of a mask and an oxygen supply device.

16. A system for providing an air quality information, comprising:
a plurality of mobile devices (1a, 1b, 1c), wherein each of the mobile devices (1a, 1b, 1c) comprises:
at least one positioning module (12) generating at least one position data; and
at least one actuating and sensing module (13) generating at least one air-sensing data;
wherein each of the mobile devices (1a, 1b, 1c) generates at least one single-point air quality data (SIa, SIb, SIc) at at least one predetermined time, and the single-point air quality data (SIa, SIb, SIc) includes the position data and the air-sensing data;
at least one cloud data processing device (2) receiving the single-point air quality data (SIa, SIb, SIc) transmitted from each of the mobile devices (1a, 1b, 1c) through a first communication transmission path, processing the single-point air quality data (SIa, SIb, SIc) of the mobile devices (1a, 1b, 1c), and generating at least one instant air quality map (21) by combining the processed single-point air quality data (SIa, SIb, SIc) with a map data (211); and
at least one client device (3) generating at least one current location data (CL) represented a current location to transmit to the cloud data processing device (2) through a second communication transmission path and sending a request for at least one information (AI) to the cloud data processing device (2), wherein the cloud data processing device (2) generates the information (AI) according to the current location data (CL) and the instant air quality map (21) and transmits the information (AI) to the client device (3).
